# EUROPEAN PATENT APPLICATION

(11) **EP 1 360 956 A1**
(43) Date of publication of application: **12.11.2003**
(21) Application number: 02732187.6
(22) Date of filing: 11.01.2002
(51) Int. Cl.: A61K 7/50

(54) **CLEANSER COMPOSITIONS**

(30) Priority: 18.01.2001 JP 2001009754
(71) Applicant: TSUMURA & CO., Chuo-ku, Tokyo 103-0027 (JP)
(72) Inventor: KARAKIDA, Fumito, TSUMURA & CO., Inashiki-gun, Ibaraki 300-1192 (JP); KOJIMA, Koji, TSUMURA & CO., Inashiki-gun, Ibaraki 300-1192 (JP)
(74) Representative: Abello, Michel
(86) International application number: JP0200111
(87) International publication number: WO02056860

(57) **Abstract**

A washing composition containing, as active ingredients, charcoal, an alkyl acrylate copolymer, and/or an α-hydroxy acid. The washing composition has a high washing effect, and it can eliminate unnecessary horny cells without giving irritation or damage to the skin, and it is also excellent in feeling in use and stability.

## Description

### Technical Field

The present invention relates to a washing composition having an excellent effect of eliminating horny cells. More specifically, the present invention relates to a washing composition, which eliminates unnecessary horny cells using charcoal, so as to prevent skin dullness generated mainly due to aging or stress from the external environment, to have excellent stability and feeling in use, and to provide a refreshing feeling with reduced soapy feeling after washing.

### Background Art

In recent years, people have been more aware of cleanliness, and thus, needs for washing agents used to keep the body clean have also been raised. Facial or body washing agents are required to have a basic function to eliminate dirt due to the adhesion of dusts or microorganisms caused by the external environment, and dirt due to sweat, sebum, waste products and others emitted from the inside of the body to the skin surface or the outside of the body. Moreover, it is also important for the facial or body washing agents not to prevent various physiological functions of the skin or not to impair the skin itself.

Disturbance in metabolic turnover has recently been pointed out as a cause of skin dullness associated with aging or stress due to changes in the external environment. Accordingly, an attempt to eliminate unnecessary horny cells using a washing agent has been made so as to reduce the disturbance in metabolic turnover, and to reduce skin dullness. Moreover, feeling in use is recognized as an important function of washing agents. It is desired for washing agents to give no soapy feeling remaining on the skin after washing, while providing a refreshing feeling.

In order to improve various functions as stated above, in particular, a function to eliminate unnecessary horny cells, the washing agents containing a scrub, a clay mineral, an inorganic powder, an organic powder, or the like have previously been developed.

However, since the particles of the above conventional washing agents, which have been mixed to eliminate keratin, have been large and hard, they have had a risk of damaging the skin during washing. Furthermore, the particles have caused a problem regarding the stability of the product in that precipitation or agglomeration has easily occurred. Still further, even though the conventional washing agents have had an excellent washing effect, soapy feeling has remained after washing and a refreshing feeling could not easily be obtained, thereby not providing fully satisfactory feeling in use.

That is to say, the actual situation is that: in the conventional washing agents, there have not been realized all the functions at a satisfactory level, such as a keratin eliminating effect with low irritation, a washing effect, and feeling in use, which have strongly been required in recent years. Moreover, problems also remain in terms of stability.

The present invention has been made to solve the above conventional problems. It is the object of the present invention to provide a washing composition having a high washing effect, being capable of eliminating unnecessary horny cells without giving irritation or damage to the skin, as well as being excellent in feeling in use and stability.

### Disclosure of the Invention

The present invention provides a washing composition characterized in that it contains, as active ingredients, charcoal and an alkyl acrylate copolymer (the first invention).

Moreover, the present invention provides a washing composition characterized in that it contains, as active ingredients, charcoal and α-hydroxy acid (the second invention).

Furthermore, the present invention provides a washing composition characterized in that it contains, as active ingredients, charcoal, an alkyl acrylate copolymer, and α-hydroxy acid (the third invention).

### Best Mode for Carrying Out the Invention

As stated above, any one of the washing compositions according to the first to third inventions contains charcoal as an active ingredient. The charcoal is contained in the washing compositions mainly to eliminate unnecessary horny cells. It is softer than a scrub agent, clay mineral, inorganic powder, organic powder, etc., which have been contained in the conventional washing compositions for the same purpose, and accordingly, it exerts an excellent keratin eliminating effect without giving excessive irritation or damage on the surface of the skin. Moreover, as a second effect, a sebum adsorbing effect and/or a deodorizing effect can also be obtained.

Furthermore, the washing compositions according to the first and third inventions contain an alkyl acrylate copolymer. The alkyl acrylate copolymer prevents the precipitation or agglomeration of charcoal in the washing composition so as to improve stability, and it also imparts excellent feeling in use to the washing composition, thereby providing a refreshing feeling after washing, while providing reduced soapy feeling. Still further, the washing compositions according to the second and third inventions contain α-hydroxy acid as a keratin dissolving agent, and the keratin eliminating effect of charcoal is improved by dissolving unnecessary horny cells on the skin using the α-hydroxy acid.

Examples of the type of charcoal contained in the washing compositions according to the first to third inventions may include those obtained by carbonizing and activating wood, sawdust, coconut husk, coal, and other materials. The mean particle size of the charcoal is preferably 0.1 to 200 µm, and particularly preferably 1 to 100 µm. If the mean particle size of the charcoal is below this range, it is difficult to obtain a sufficient keratin eliminating effect, but if it exceeds this range, the user has an uncomfortable feeling during washing. The content of the charcoal is preferably 0.01 to 3% by mass, and further preferably 0.05 to 1% by mass. If the content of the charcoal in the washing composition is below this range, it is difficult to obtain a sufficient keratin eliminating effect, but if it exceeds this range, stability and feeling in use deteriorate during washing.

The type of an alkyl acrylate copolymer contained in the washing compositions according to the first and third inventions is not particularly limited. For example, there can be used singly or in combination of two or more types of; an ethyl acrylate-butyl acrylate copolymer, an ethyl acrylate-methacrylic acid copolymer, an ethyl acrylate-methyl methacrylate copolymer, an ethyl acrylate-ethyl methacrylate copolymer, a butyl acrylate-ethyl methacrylate copolymer, an ethyl acrylate-ethyl methacrylate-acrylic acid copolymer, a methacrylic acid-ethyl acrylate-butyl acrylate copolymer, an octyl acrylate-styrene copolymer, an octyl acrylate-vinyl acetate copolymer, a methoxyethyl acrylate-hydroxyethyl acrylate copolymer, a butyl acrylate-ethyl hydroxymethacrylate copolymer, a methoxyethyl acrylate-hydroxyethyl acrylate-butyl acrylate copolymer, and a hydroxypropyl acrylate-butylaminoethyl methacrylate-octylamide acrylate copolymer, and the like. The content of the alkyl acrylate copolymer is preferably 0.01 to 5% by mass, and further preferably 0.05 to 3% by mass. If the content of the alkyl acrylate copolymer in the washing composition is below this range, an effect of preventing the precipitation or agglomeration of charcoal is reduced, and thus, it becomes difficult to obtain sufficient stability. In contrast, if the content exceeds this range, it becomes difficult to obtain satisfactory feeling in use.

The type of α-hydroxy acid contained in the washing compositions according to the second and third inventions is not particularly limited. For example, there can be used singly or in combination of two or more types of; lactic acid, citric acid, malic acid, glycolic acid, α-hydroxyoctanoic acid, α-hydroxypalmitic acid, α-hydroxystearic acid, and alkali salts thereof and the like. The content of the α-hydroxy acid is preferably 0.001 to 20% by mass, and further preferably 0.01 to 10% by mass. If the content of the α-hydroxy acid is below this range, a keratin dissolving effect is reduced. However, even if the content exceeds this range, a greater effect resulting from the increased amount cannot be obtained, and stability deteriorates.

In addition to the above described active ingredients, various types of ingredients used in common washing agents can be added to the washing composition of the present invention, as necessary. Examples of such ingredients include surfactant; fatty acid; melanogenesis inhibitor; antioxidant; plant extract having a whitening effect such as Mulberry bark (Mori Cortex), Aloe(Aloe), Rose Fruit (Rosae Fructus), Sweet Hydrangea Leaf (Hydrangeae Dulcis Folium), Almond (Prunus amygdalus Batsch), Ginkgo (Ginkgo biloba L.), Oolong Tea (Thea sinensis L.), Scutellaria Root (Scutellariae Radix), Japanese Coptis Rhizome (Coptidis Rhizoma), Hypericum (Hypericum perforatum L.), Pueraria Root (Puerariae Radix), Gardenia Fruit (Gardeniae Fructus), Sophora Root (Sophorae Radix), Rice bran (Oryza sativa L.), Asiasarum Root (Asiasari Radix), Zanthoxylum Fruit (Zanthoxyli Fructus), Beefsteak Leaf (Perillae Herba), Soap Root (Saponariae Radix), Peony Root (Paeoniae Radix), Cnidium Rhizome (Cnidii Rhizoma), Glycyrrhiza (Glycyrrhizae Radix), Soybean (Glycine max Merril), Green Tea (Theae Folium), Japanese Angelica Root (Asparagi Radix), Calendula (Calendula officinalis L.), Garlic (Allii Bulbus), Witch Hazel (Hamamelis virginiana L.), Sponge Cucumber Gourd(Luffa cylindrica M. Roemen), Tree Peony (Moutan Cortex), Balm Mint (Melissa officinalis L.), Eucalyptus (Eucalyptus globulus Labillardiere or other species of the same genus), Coix Seed (Coicis Semen), Lemon (Citrus limonum Risso), Rosemary (Rosemarinus officinalis L.), Burnet (Sanguisorba officinalis L.) or Black Tea (Thea sinensis L.); vitamin C and derivative thereof; etc. The washing composition of the present invention containing the above active ingredients can preferably be used as a washing composition for the skin or the elimination of keratin.

The present invention will be described further in detail in the following examples. The examples are not intended to limit the scope of the invention.

### (Examples 1 to 4, and Comparative Examples 1 to 4)

According to a conventional method, body soap with the composition as shown in Table 1 was produced, and its stability, keratin eliminating effect and feeling in use were evaluated. The results of the evaluation are shown in the same table. The method of carrying out each evaluation is as follows:

### (1) Evaluation of stability

Each washing agent was placed in a transparent container, and it was conserved in a constant temperature bath at 40°C for 3 months. Thereafter, the appearance was observed by the naked eye, and the evaluation was carried out in accordance with the following standards:
○: No change is observed.
Δ: Separation and precipitation are slightly observed.
×: Separation and precipitation are considerably observed.

### (2) Evaluation of keratin eliminating effect

Using a spectrophotometer, the luminosity (L1) of the measurement part of the antebrachium was measured. Washing treatment was carried out thereon using each washing agent. Thereafter, the antebrachium was dried, and the luminosity (L2) of the treatment part was measured again. The difference between L1 and L2 (color difference) was obtained, and the evaluation was carried out in accordance with the following standards:
ⓞ: 2.0 or more
○: 1.5 to less than 2.0
Δ: 1.0 to less than 1.5
× : less than 1.0

### (3) Evaluation of feeling in use (soapy feeling, refreshing feeling)

Fifty specialized panelists were asked to use each of the washing agents to determine the soapy feeling and refreshing feeling of the skin after washing on a scale of 1 to 5. Thereafter, the mean values were calculated, and the evaluation was carried out in accordance with the following standards:
○: 5.0 to 4.0
Δ: 3.9 to 3.5
×: less than 3.5

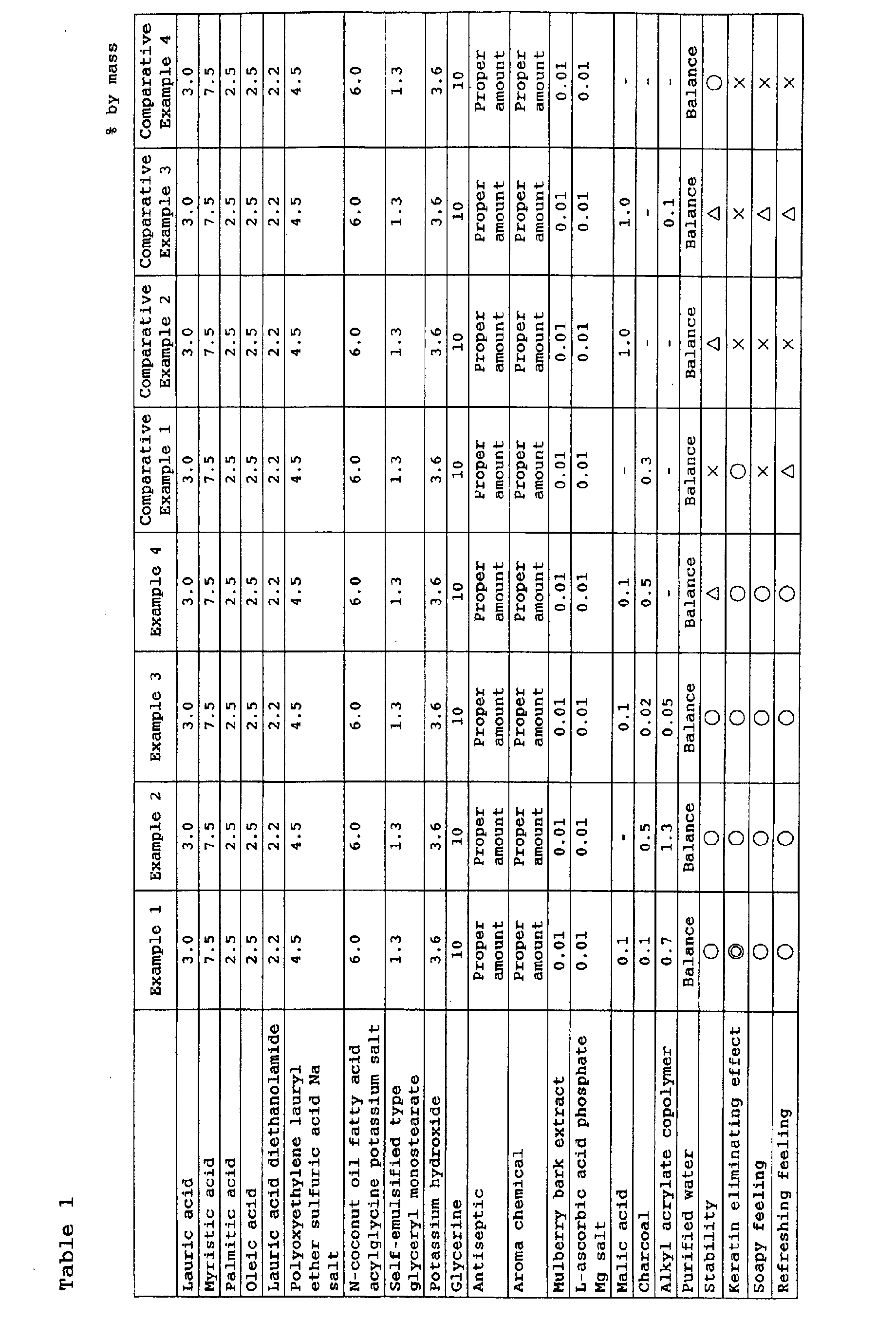

### (Example 5)

According to a conventional method, a facial soap with the following composition was produced, and its washing effect, stability, keratin eliminating effect and feeling in use were evaluated. The facial soap had excellent results in any of the above evaluation points.

| | |
|---|---|
| Stearic acid | 10.3% by mass |
| Palmitic acid | 9.8% by mass |
| Myristic acid | 11.6% by mass |
| Lauric acid | 5.2% by mass |
| Lauric acid amide propylbetaine | 2.6% by mass |
| Lauric acid diethanolamide | 3.1% by mass |
| Polyethylene glycol | 10.1% by mass |
| Glycerine | 12.0% by mass |
| 1,3-butylene glycol | 2.0% by mass |
| N-acylglutamic acid Na salt | 3.0% by mass |
| Potassium hydroxide | 5.9% by mass |
| Ascorbic acid | 0.1% by mass |
| Saxifrage extract | 0.1% by mass |
| Charcoal | 0.5% by mass |
| Alkyl acrylate copolymer | 1.0% by mass |
| Lactic acid | 0.1% by mass |
| Paraben | 0.1% by mass |
| Aroma chemical | proper amount |
| Sequestering agent | proper amount |
| Purified water | balance |

### (Example 6)

According to a conventional method, body soap with the following composition was produced, and its washing effect, stability, keratin eliminating effect and feeling in use were evaluated. The body soap had excellent results in any of the above evaluation points.

| | |
|---|---|
| Myristic acid | 23.3% by mass |
| Stearic acid | 4.7% by mass |
| Lauric acid diethanolamide | 2.3% by mass |
| POE coconut oil fatty acid monoethanolamide sulfuric acid Na salt | 4.9% by mass |
| POE lauryl ether sulfuric acid Na salt | 4.9% by mass |
| N-coconut oil fatty acid acylglycine K salt | 2.0% by mass |
| Glyceryl monostearate | 1.6% by mass |
| Glycerine | 13.1% by mass |
| Potassium hydroxide | 5.9% by mass |
| Sodium hydroxide | 0.1% by mass |
| 1,3-butylene glycol | 6.5% by mass |
| L-ascorbic acid phosphate Mg salt | 0.01% by mass |
| Mulberry bark extract | 0.1% by mass |
| Charcoal | 0.1% by mass |
| Alkyl acrylate copolymer | 0.7% by mass |
| Malic acid | 0.1% by mass |
| Black iron oxide | 0.3% by mass |
| Talc | 0.2% by mass |
| Paraben | 0.1% by mass |
| Edetic acid disodium salt | 0.1% by mass |
| Aroma chemical | proper amount |
| Purified water | balance |

### (Example 7)

According to a conventional method, body soap with the following composition was produced, and its washing effect, stability, keratin eliminating effect and feeling in use were evaluated. The body soap had excellent results in any of the above evaluation points.

| | |
|---|---|
| Stearic acid | 11.0% by mass |
| Palmitic acid | 7.9% by mass |
| Myristic acid | 12.1% by mass |
| Lauric acid | 3.9% by mass |
| Oleyl alcohol | 1.5% by mass |
| Purified lanolin | 1.0% by mass |
| Coconut oil fatty acid diethanolamide | 1.0% by mass |
| Glycerine | 10.0% by mass |
| Potassium hydroxide | 10.0% by mass |
| Aloe extract | 0.1% by mass |
| Charcoal | 0.05% by mass |
| Alkyl acrylate copolymer | 0.3% by mass |
| Glycolic acid | 0.1% by mass |
| Paraben | 0.1% by mass |
| Aroma chemical | proper amount |
| Sequestering agent | proper amount |
| Purified water | balance |

### (Example 8)

According to a conventional method, hand soap with the following composition was produced, and its washing effect, stability, keratin eliminating effect and feeling in use were evaluated. The hand soap had excellent results in any of the above evaluation points.

| | |
|---|---|
| Stearic acid | 8.0% by mass |
| Palmitic acid | 2.0% by mass |
| Myristic acid | 6.0% by mass |
| Lauric acid | 2.0% by mass |
| Lauryl dimethyl aminoacetic acid betaine | |
| 4.0% by mass | |
| Propylene glycol | 8.0% by mass |
| Triclosan | 0.3% by mass |
| Potassium hydroxide | 5.0% by mass |
| Oil-soluble liquorice root extract | 0.1% by mass |
| Oolong | 0.1% by mass |
| Charcoal | 0.1% by mass |
| Alkyl acrylate copolymer | 0.2% by mass |
| Malic acid | 0.1% by mass |
| Paraben | 0.1% by mass |
| Aroma chemical | proper amount |
| Sequestering agent | proper amount |
| Purified water | balance |

### (Example 9)

According to a conventional method, a solid soap with the following composition was produced, and its washing effect, stability, keratin eliminating effect and feeling in use were evaluated. The solid soap had excellent results in any of the above evaluation points.

| | |
|---|---|
| Higher fatty acid sodium salt | 80.0% by mass |
| Glycerine | 10.0% by mass |
| Edetic acid | 0.1% by mass |
| Birch bark (Betula Alba) extract | 0.1% by mass |
| Charcoal | 3.0% by mass |
| Alkyl acrylate copolymer | 3.0% by mass |
| Citric acid | 0.5% by mass |
| Paraben | 0.1% by mass |
| Aroma chemical | proper amount |
| Sequestering agent | proper amount |
| Purified water | balance |

### Industrial Applicability

As stated above, the washing composition of the present invention can effectively eliminate unnecessary horny cells that cause skin dullness, without giving excessive irritation or damage on the surface of the skin. Moreover, the washing composition has excellent stability and feeling in use, and further, it provides a refreshing feeling with reduced soapy feeling after washing.

## Claims

1. A washing composition **characterized in that** it contains, as active ingredients, charcoal and an alkyl acrylate copolymer.

2. A washing composition **characterized in that** it contains, as active ingredients, charcoal and an α-hydroxy acid.

3. A washing composition **characterized in that** it contains, as active ingredients, charcoal, an alkyl acrylate copolymer, and an α-hydroxy acid.

4. The washing composition according to any one of claims 1 to 3, which is used for the skin.

5. The washing composition according to any one of claims 1 to 3, which is used for elimination of keratin.
